(19) Europäisches Patentamt
European Patent Office
Office européen des brevets

(11) Publication number: **0 052 910**
**B1**

(12) # EUROPEAN PATENT SPECIFICATION

(45) Date of publication of patent specification: **10.04.85**

(51) Int. Cl.[4]: **C 07 C 153/07,**
C 07 C 129/12, C 07 C 101/24,
C 07 H 17/08, A 61 K 31/265

(21) Application number: **81201309.2**

(22) Date of filing: **25.11.81**

(54) **Derivative of N-acetilcysteine having therapeutical activity, process for its preparation and related pharmaceutical compositions.**

(30) Priority: **26.11.80 IT 2623380**

(43) Date of publication of application:
**02.06.82 Bulletin 82/22**

(45) Publication of the grant of the patent:
**10.04.85 Bulletin 85/15**

(84) Designated Contracting States:
**BE DE FR GB**

(56) References cited:
**EP-A-0 008 833**
**CH-A- 274 379**
**US-A-2 488 253**
**US-A-4 072 703**

**O.A. NEUMÜLLER "Römpps Chemie Lexikon", 8th edition, vol. 1, A-Cl, 1979, FRANCKH'SCHE VERLAGSHANDLUNG, Stuttgart, page 44**
**"ULLMANNS ENCYKLOPÄDIE DER TECHNISCHEN CHEMIE, 4th edition, vol. 7, 1974, VERLAG CHEMIE, Weinheim/Bergstrasse, page 547**

**The file contains technical information submitted after the application was filed and not included in this specification**

(73) Proprietor: **Real S.a.s. di Alberto Reiner & C.**
**Via Cattaneo, 1**
**I-22063 Cantù (Como) (IT)**

(72) Inventor: **Reiner, Alberto**
**Via Mentana, 23**
**Como I (IT)**

(74) Representative: **Dragotti, Gianfranco**
**SAIC BREVETTI S.a.s. di Ing. G. Dragotti & C. Via Spontini, 1**
**I-20131 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 052 910 B1**

## Description

The present invention relates to a thioester of N-acetylcysteine propionylglicine with p-isobutylphenylpropionic acid and having the formula:

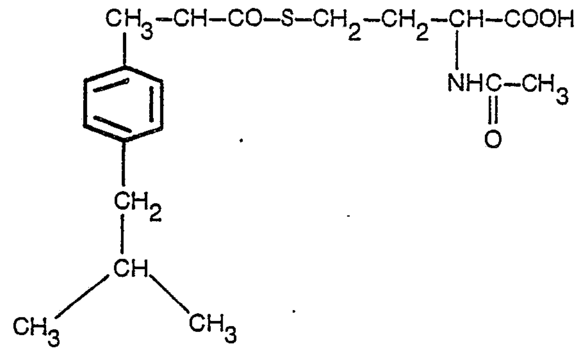

having interesting anti-inflammatory and mucolytic properties, as well as to its non toxic and pharmacologiocally acceptable salts. The anti-inflammatory activity of the p-isobutylphenylpropionic acid is known, and there are also known its disadvantages from the therapeutical and use point of view.

The compound of the invention, as such or converted to a salt with inorganic bases containing alkaline or earth-alkaline ions, or with basic aminoacids, such as arginine ad lysine, or with nuclei of basic antibiotics, such as erythromycin and propionylerythromycin, shows interesting properties, since it is simultaneously endowed with anti-inflammatory and mucolithic activity.

The present invention relates also to the process for the preparation of the subject compound, which is characterized in that a halide of p-isobutylphenylpropionic acid and a derivative of N-acetylcysteine are reacted in a medium having alkaline pH comprising a polar solvent, preferably water or mixtures of water and dioxane, at low temperature, giving place to a low melting thioester which, at room temperature, is an odorless pale yellow oil. The reaction product, as such is devoid of impurities and it utilized in the salt forming reaction with the several bases above referred to, in order to obtain the corresponding salts, which are stable, water soluble and easy to be used in the pharmaceutical field.

The following example illustrates, in non limiting sense, the process for the preparation of the derivative of the invention.

Example

A flask is charged with 600 mls of chloroform and 250 mls of $SOCl_2$ and this solution is slowly supplemented with 500 g of p-isobutylphenylpropionic acid. The latter is dissolved by lowering the temperature to a value near 0°C. Then a heating to 20—25°C is carried out and at that point $SO_2$ and HCl are developed. The heating is slowly continued up to 60—63°C, the reaction being monitored on the basis of the gas development and until the latter ceases. The reaction mixture is concentrated under vacuum to eliminate the solvent and the unreacted thionyl chloride, and an oil is obtained which is used as such for the subsequent reaction. There are obtained 540 g of the chloride of the p-isobutylphenylpropionic acid.

b) preparation of thioester of p-isobutylphenylpropionic of N-acetylcysteine

A flask is charged with 50 mls of water: after a cooling to a temperature of about 0°C, 16.32 g (0.1 moles) of N-acetyl-(L)-cysteine and 4 g of a 50% solution of NaOH are added. The reaction mixture is maintained under cooling at 0°C and there are slowly and simultaneously added 22.5 g of chloride of the p-isobutylphenylpropionic acid and 4 g of NaOH dissolved in 20 mls of water. The reaction during this addition is hexothermic and the temperature is controlled so that it is not higher than +5°C. The reaction time is of between 3 and 5 hours. Upon the reaction is completed, the temperature is increased and then the volume is doubled with further water, the reaction mixture being then made acidic with HCl until a definitely acid pH is obtained.

The oily product is extracted with methylisobutylketone, the extract is dried over $Na_2SO_4$, it is concentrated to dryness and a dense oil is obtained (yield: 32 g). The oil is dissolved in isopropyl alcohol, is filtered with carbon and concentrated again to dryness. A very dense oily product is obtained, which is chromatographically pure.

c) preparation of the salts of erythromycin and propionylerythromycin of p-isobutylphenylpropionoylthio-N-acetylcysteine

These salts are readily obtained in solvents of the classes of ketones and alcohols by reacting one mole of erythromycin base or propionylerythromycin with one mole of p-isobutylphenylpropionoylthio-N-acetylcysteine. The products are white, low melting crystals.

The products of the present invention show mucolytic activity, besides the anti-inflammatory activity, and can be used in all the possible pharmaceutical forms, such as tablets, capsules, syrups, aerosols, normal or lyophilized vials, granulated formulations, creams, etc.

The thioester of N-acetyl-cysteine with p-isobutylphenylpropionic acid has been pharmacologically tested, with the following results:

1) Toxicity

It has not been possible to determine the $LD_{50}$ since at the dose of 900 mg/kg no cases of death were revealed.

2) Anti-inflammatory activity

The tests used for the evaluation of the anti-inflammatory activity were respectively that of the kaolin and that of the egg white.

a) Anti-inflammatory activity in the kaolin test. For each compound 16 rats of male sex, of the Sprague-Dawley strain and of 200—250 g of body weight, were used. The rats were divided into four groups each comprising four rats. All the animals were injected with a 10% suspension of kaolin. The first group was used as the control, whereas the other three groups were administered with doses respectively of 50, 75 and 100 mg/kg. The measurements of the diameters of the articulation were effected one hour after the kaolin injection and then every 24 hours.

The obtained data are reported in the table 1. The inoculation of kaolin caused an increase of the tibio-tarsal articulation which has been partially limited by the treatment with the test compound.

TABLE 1

Kaolin test

| DOSES | Anti-inflammatory activity % |
|---|---|
| Controls | |
| (Kaolin) | — |
| 50 mg/kg | — |
| 75 mg/kg | 12 |
| 100 mg/kg | 26 |

b) Egg white test.

For each compound 16 male rats, of the Sprague-Dawley strain and weighing 300 g, were used. The animals were divided into four groups each comprising four rats. The first group served as a control, whereas the other three groups were orally administered, with a 30 minute interval, with two doses of 50, 75 and 100 mg/kg of the test compound.

The last administration was immediately followed by the inoculation of 0.1 ml of egg white in the rear paw of all the animals, including the controls.

The measurements of the diameters of the articulations were effected 3 and 4 hours later.

The data obtained in the present test are reported in the table 2.

TABLE 2

Egg white test

| DOSES | Anti-inflammatory activity % |
|---|---|
| Controls | — |
| 50 mg/kg | — |
| 75 mg/kg | 12 |
| 100 mg/kg | 20 |

The thioester of N-acetylcysteine with p-isobutylhenylpropionic acid is formulated according to the present invention and the aforesaid salts are formulated by the known techniques, excipients, solvents, fillers, etc., giving place to pharmaceutical compositions in form of tablets, suppositories, injectable solutions, suspensions, syrups, emulsions, creams and aerosols.

In fact the stability and solubility of the subject compound and of its salts allow for such a complete range of uses.

**Claims**

1. Thioester of N-acetylcysteine with p-isobutylphenylpropionic acid and the non toxic and pharmaceutically acceptable salts thereof with organic and inorganic bases.

2. Thioester according to claim 1, characterized in that said salts are formed with inorganic bases containing alkaline and earth-alkaline ions.

3. Thioester according to claim 1, characterized in that said salts are formed with basic aminoacids.

4. Thioester according to claim 3, characterized in that said basic aminoacids are arginine and lysine.

5. Thioester according to claim 1, characterized in that said salts are formed with basic antibiotics.

6. Thioester according to claim 5, characterized in that said basic antibiotics are erythromycin and propionylerythromycin.

7. A process for the preparation of the thioester of N-acetyl-cysteine with p-isobutylpropionic acid, characterized in that a halide of the p-isobutylphenylpropionic acid is reacted with a derivative of N-acetylcysteine in a polar medium at an alkaline pH and at low temperature.

8. A process according to claim 7, characterized in that said halide is the chloride and said derivative of N-acetylcysteine is the sodium salt.

9. A process according to claim 7, characterized in that said polar medium comprises water or mixtures of water and dioxane and the reaction temperature is of between 0 and 5°C.

10. Pharmaceutical composition having anti-inflammatory and mucolytic activity, characterized by containing together with the common excipients and/or solvents, an effective amount of the thioester of N-acetyl-cysteine with p-isobutylphenylpropionic acid or a non toxic and pharmacologically acceptable salt thereof according to claims 1 to 6.

11. Pharmaceutical composition according to claim 10, in form of tablets, syrups and suspensions for the oral administration.

12. Pharmaceutical composition according to claim 10, in form of solution of the parenteral administration.

13. Pharmaceutical composition according to claim 10, in form of suppositories for the rectal administration.

14. Pharmaceutical composition according to claim 10, in form of cream, ointment, aerosol and the like for topical administration.

**Patentansprüche**

1. Thioester von N-acetylcystein mit p-Isobutylphenylpropionsäure und die nicht toxischen und pharmazeutisch annahmbaren Salze davon mit organischen und anorganischen Basen.

2. Thioester nach Anspruch 1, dadurch gekennzeichnet, daß die genannten Salze mit alkalische und erdalkalische Jonen enthaltenden anorganischen Basen gebildetet sind.

3. Thioester nach Anspruch 1, dadurch gekennzeichnet, daß die genannten Salze mit basischen Aminosäuren gebildet sind.

4. Thioester nach Anspruch 3, dadurch gekennzeichnet, daß die genannten basischen Aminosäuren Arginin und Lysin sind.

5. Thioester nach Anspruch 1, dadurch gekennzeichnet, daß die genannten Salze mit basischen Antibiotika gebildet sind.

6. Thioester nach Anspruch 1, dadurch gekennzeichnet, daß die genannten basischen Antibiotika Erythromycin und Propionylerythromycin sind.

7. Ein Verfahren zur Herstellung des Thioesters von N-acetylcystein mit p-Isobutylphenylpropionsäure, dadurch gekennzeichnet, daß ein Halid der p-Isobutylphenylpropionsäure in einem polaren Medium bei einem alkalischen pH und bei einer niedrigen Temperatur mit einem N-acetylcystein derivat umgesetzt wird.

8. Ein Verfahren nach Anspruch 7, dadurch gekennzeichnet, daß das Halid das Chlorid ist und daß das Derivat von N-acetylcystein das Natriumsalz ist.

9. Ein Verfahren nach Anspruch 7, dadurch gekennzeichnet, daß das genannte polare Medium Wasser und Wasser-Dioxan-gemische umfasst und daß die Reaktionstemperatur zwischen 0°C und 5°C liegt.

10. Pharmazeutische Zusammensetzung mit anti-inflammatorischer und mucolytischer Wirksamkeit, dadurch gekennzeichnet, daß sie zusammen mit den üblichen Bindemitteln und/oder Lösungsmitteln eine effektive Menge des Thioesters von N-acetylcystein mit p-Isobutylphenylpropionsäure oder ein nicht toxisches und pharmakologisch annahmbares Salz davon nach den Ansprüchen 1 bis 6 enthält.

11. Pharmazeutischen Zusammensetzung nach Anspruch 10, in Form von Tabletten, Syrups und Aufschlämmungen zur oralen Verabreichung.

12. Pharmazeutischen Zusammensetzung nach Anspruch 10, in Form einer Lösung zur parenteralen Verabreichung.

13. Pharmazeutische Zusammensetzung nach Anspruch 10, in Form von Suppositorien zur rectalen Verabreichung.

14. Pharmazeutische Zusammensetzung nach Anspruch 10, in Form einer Creme, Salbe, Aerosol und dgl zur topicalen Verabreichung.

**Revendications**

1. Thioester de N-acétylcysteine avec l'acide isobutylphenylpropionique et ses sels non toxiques et pharmaceutiquement acceptables avec des bases organiques et inorganiques.

2. Thioester selon la révendication 1, caractérisé en ce que les dites sels sont formés avec des bases inorganiques qui contienent des ions alcalins et alcalin-terreux.

3. Thioester selon la révendication 1, caractérisé en ce que les dites sels sont formés avec des aminoacides basiques.

4. Thioester selon la révendication 1, caractérisé en ce que les dites aminoacides basiques sont arginine et lysine.

5. Thioester selon la révendication 1, caractérisé en ce que les dites sels sont formés avec antibiotiques basiques.

6. Thioester selon la révendication 5, caractérisé en ce que les dites antibiotiques basiques sont erythromycine et propionylerythromycine.

7. Un procédé pour la préparation du thioester de N-acétylcysteine avec l'acide p-isobutylphenylpropionique, caractérisé en ce qu'on fait reagir un halogénure de l'acide p-isobutylphenylpropionique avec un dérivé de l'N-acétylcysteine dans un milieu polaire à un pH alcalin et à une faible température.

8. Un procédé selon la révendication 7, caractérisé en ce quye le dit halogénure est le chlorure et le dit dérivé de N-acétylcysteine est le sel de sodium.

9. Un procédé selon la révendication 7, caractérisé en ce que le dit milieu polaire comporte l'eau on des melanges d'eau et de dioxane et la témperature de reaction est comprise entre 0 et 5°C.

10. Composition pharmaceutique ayant activité anti-inflammatoire et mucolitique, caractérisé en ce qu'elle contient, avec les excipients et/ou solvants communs, une quantité efficace du thioester de l'N-acétylcysteine avec l'acide p-isobutylphenylpropionique ou un de ses sels non toxique et pharmacologiquement acceptable selon les revendications 1 à 6.

11. Composition pharmaceutique selon la révendication 10, sous forme de comprimés, sirops et suspensions pour l'administration orale.

12. Composition pharmaceutique selon la revendicaation 10, sous forme de solution pour l'administration parentérale.

13. Composition pharmaceutique selon la révendication 10, sous forme de suppositoires pour l'administration rectale.

14. Composition pharmaceutique selon la revendicaation 10, sous forme de crème, onguent aerosol et semblables pour l'administration topique.